(19) Europäisches Patentamt
European Patent Office
Office européen des brevets

(11) Publication number: **0 139 423**
**A2**

(12) # EUROPEAN PATENT APPLICATION

(21) Application number: 84305948.6

(22) Date of filing: 30.08.84

(51) Int. Cl.[4]: C 01 B 3/58
C 01 B 3/26, C 07 C 31/04
C 07 C 29/15

(30) Priority: 12.09.83 DK 4119/83

(43) Date of publication of application:
02.05.85 Bulletin 85/18

(84) Designated Contracting States:
BE DE FR GB IT NL

(71) Applicant: Haldor Topsoe A/S
Nymollevej 55
DK-2800 Lyngby(DK)

(72) Inventor: Adriansen, Jorgen
Aldersrovej 17
DK-2950 Vedbaek(DK)

(72) Inventor: Christiansen, Claus
Naerum Hovegade 76
DK-2850 Naerum(DK)

(74) Representative: Kyle, Diana et al,
ELKINGTON AND FIFE High Holborn House 52/54 High
Holborn
London WC1V 6SH(GB)

(54) **A process for the preparation of high-purity hydrogen.**

(57) In the production of hydrogen by the catalytic decomposition of methanol and the subsequent purification thereof by removing carbon oxides inevitably formed as a by-product by catalytically converting them into methanol, optionally after a preliminary removal by other means, an improved overall economy is obtained by recycling the methanol formed so as to combine it with the feedstock of methanol.

Croydon Printing Company Ltd.

A PROCESS FOR THE PREPARATION OF HIGH-PURITY HYDROGEN

The present invention relates to a process for the preparation of hydrogen having a degree of purity, by the removal of carbon oxides form a hydrogen-containing gas prepared by the catalytic decomposition of methanol.

Hydrogen having a high degree of purity is a technically important product which is produced in very big annual amounts, partly at the locality of use and partly centrally for distribution. Hydrogen is used within the organic and inorganic synthesis industries, e.g. for catalytic hydrogenations and in metallurgy, and for many other purposes.

In some commercially employed processes for producing hydrogen the hydrogen is obtained in a pure or almost pure form but, for example,in the process relevant in the present context, the catalytic decomposition of methanol , which is a technically important process, the hydrogen formed is inevitably accompanied by carbon oxides.

The purification of such a hydrogen-containing gas mixture in order to obtain hydrogen having a high degree of purity therefore is of technical importance and is well-known *per se*.

Although the purification process is an extra step in the production process and may be comparatively complicated because it must often comprise several successive partial process steps to provide sufficiently pure hydrogen, the production of hydrogen having a high degree of purity by the purification of a hydrogen-containing gas mixture will nevertheless often be economically advantageous in comparison with the production

of hydrogen in a manner which directly gives hydrogen having a high degree of purity. It is, however, important to combine the production and the purification of hydrogen in a manner to achieve the lowest possible overall cost.

Carbon dioxides (CO and $CO_2$) are impurities in hydrogen that will cause drawbacks in many used. The drawbacks may consist in a poisoning of the catalysts which are used, e.g., in technically important hydrogenations; or in the formation of undesirable by-products in the processes aimed at. The drawbacks may also be merely a dilution of the hydrogen resulting in a decrease of the reaction rate of the desired processes. This may be counteracted by purging the impurities as they are accumulated during the process but inevitably this will also cause a loss of hydrogen.

Several processes for the removal of CO and/or $CO_2$ from hydrogen-containing gas mixtures are known.

Processes for the removal of CO from a gas mixture are, among others:

Washing with a solution of complex copper salts. This process is expensive, gives big operating difficulties and is seldom used in modern plants.

Conversion of CO with steam into $CO_2$ and $H_2$ in the presence of a catalyst by the so called shift reaction. The process is commonly used for a preliminary purification to remove most of the CO but imparts very heavy operating costs if a particularly low residual content of CO is to be achieved.

Removal of CO from hydrogen by a selective catalystic oxidation into $CO_2$ while adding oxygen. The process leaves small amounts of oxygen in the purified gas, which may be undesirable for many uses.

Cyrogenic distillation, which is a process which imparts heavy operating costs.

Processes for the removal of $CO_2$ from a gas mixture are, among others:

Washing out with water or special solvents. The process is commonly used for a preliminary purification to remove most of the $CO_2$ but imparts heavy operating costs if a particularly low residual amount of $CO_2$ is to be achieved.

Selective adsorption whereby very pure hydrogen can be prepared, but only with outputs of about 75-90% and in apparatus requiring disproportionately large investments.

Processes for the removal of both CO and $CO_2$ from a gas mixture are, among others:

Adjustment of the ratio of $CO_2$ to $H_2$ and possibly also CO to $H_2$ while using membranes. The process can only be used to adjust the proportions mentioned and not to provide hydrogen of a high degree of purity under industrial conditions and at a high output.

Diffusion of $H_2$ through Pd or other metals or alloys. The process yields extremely pure hydrogen, is especially employed in small hydrogen plants because of the costs, for example, Pd-cells, and the output is comparatively low.

Washing with liquid $N_2$. The process can remove the majority of undesirable components from the hydrogen to a high degree of completeness, but adds $N_2$ to the hydrogen and imparts big operating costs.

Catalytic conversion of CO and $CO_2$ into $CH_4$. Each mol of CO and/or $CO_2$ yields one mol of $CH_4$, and hence the hydrogen does not become more pure and is no easier to purify; but it is often more usable than hydrogen containing CO and/or $CO_2$.

It is thus characteristic of these known processes that they impart very heavy operating costs if the residual content of CO and/or $CO_2$ must be brought to a very low level, and/or impart to the hydrogen

a corresponding increase of the contents of other gases, and/or give a limited output.

From German patent publication (OLS) No. 1,902,107 it is known to remove carbon oxide from hydrogen-rich gas mixture by reducing the carbon oxide to methanol and removing the latter from the gas mixture. This method of removing the carbon oxide from the hydrogen-rich mixture is not related to the manner of producing the hydrogen-rich gas mixture, and the only origin of this mentioned in the specification is coke oven gases. Though not positively stated, it seems that only carbon monoxide, CO, may be present in the hydrogen-rich gas to be purified since carbon dioxide is not mentioned. The reduction of the carbon oxide according to the publication may be carried out at 100-300°C and at pressures - depending of the ultimate use of the hydrogen - of 120-500 atm. The methanol formed is removed by cooling to a temperature below its dew point, whereby the condensed methanol can easily be removed in a separator. Small residues possibly left may be removed by washing with water or by adsorption from the gas.

In itself this manner of removing the carbon oxide is advantageous but it has now been found that the overall economy of the production and purification of hydrogen can be improved significantly in a manner not foreseen in the German patent publication No. 1,902,107 if the production of the hydrogen is carried out by the catalytic decomposition of methanol and combined with the removal of the carbon oxides formed as a by-product.

Thus, the present invention provides a process for the removal of carbon oxides from a hydrogen-containing gas prepared by the catalytic decomposition of methanol, wherein a preliminary purification of the hydrogen-containing gas thereby produced is carried out

partially to remove carbon oxides therefrom, after which the hydrogen-containing gas with a reduced carbon oxides content thus obtained is passed at a temperature of 100-300°C, and a pressure of 5-300 kg/cm$^2$ gauge, preferably 15-150 kg/cm$^2$ gauge over a catalyst having activity for converting carbon oxides into methanol and the methanol formed is removed from the gas; and wherein the methanol formed is recycled and combined with the methanol used as raw material for preparing the hydrogen-containing gas.

The invention is illustrated with reference to the accompanying drawing which is a flow diagram schematically showing a preferred manner of combining the decomposition of methanol to form a hydrogen-containing gas, and removing and recycling carbon oxides formed as by-products from the resulting gas mixture.

An inlet stream 1 mainly consisting of gaseous methanol and steam is introduced into a heated reactor 2 containing a catalyst for decomposing methanol according to the reactions:

1) $CH_3OH \longrightarrow CO + 2H_2$

2) $CH_3OH + H_2O \longrightarrow CO_2 + 3H_2$

At the same time the shift reaction takes place and runs to equilibrium:

3) $CO + H_2O \rightleftarrows CO_2 + H_2$

The outlet stream 3 from reactor 2 is cooled and thereafter passed into an adiabatic reactor 4 containing a catalyst ensuring that equilibrium is obtained for the shift reaction 3) (the equilibrium is different at the lower temperature than that in reaction 2) whereby the content of CO is decreased further because of the lower temperature.

The outlet stream 5 from reactor 4 is passed into a washing tower 6 where unreacted methanol is washed out with a stream of water 22 and together with this water is recycled to the inlet of reactor 2 as a recylce stream 7. The outlet stream 8 from washing tower 6 is passed into a washing tower 9 where $CO_2$ is washed out as a stream 10. The outlet stream 11 from washing tower 19 is passed into an adiabatic reactor 12 containing a catalyst for the reactions:

4) $CO + 2H_2 \longrightarrow CH_3OH$

5) $CO_2 + 3H_2 \longrightarrow CH_3OH + H_2O$

the outlet stream 13 from reactor 12 is cooled and passed into a washing tower 14 wherein the methanol formed in reactor 12 is washed out with a stream of water 23 and thereafter together with the water recycled to the inlet of reactor 2 as a recycle stream 15. The outlet stream 16 from the washing tower 14 after drying contains 99.9 mol% $H_2$ and less than 0.1 mol% $CO + CO_2$ and in some cases may be used without further purification. In those cases where the content of impurities in the outlet stream 16 cannot be tolerated the outlet stream 16 is purified further by passage through a reactor 17 containing a catalyst for the reactions:

6) $CO + 3H_2 \longrightarrow CH_4 + H_2O$

7) $CO_2 + 4H_2 \longrightarrow CH_4 + 2H_2O$

The outlet stream 18 from reactor 17 after cooling and separation of condensate contains 99.88 mol% $H_2$, 0.05 mol% $CH_4$ and 0.07 mol% $H_2O$ and will be suitable for the majority of uses. If the content of $H_2O$ is undesired the outlet stream 18 is purified further by

passage though an adsorption drier 19 after which the product gas 20 contains 99.95% $H_2$ and 0.05% $CH_4$.

It is a characteristic feature of the process of the invention that the total content of CO and $CO_2$ in the hydrogen-containing gas is decreased substantially, these impurities being converted by a catalytic process which has been known since the beginning of the 20th century. The methanol formed may be easily removed, e.g. by being washed out with water, and recycled to the methanol feedstock from which the hydrogen has been prepared.

The predominant reactions in this catalytic process are:

4) $CO + 2H_2 \longrightarrow CH_3OH$

5) $CO_2 + 3H_2 \longrightarrow CH_3OH + H_2O$

The reactions are equilibrium reactions which means that neither CO nor $CO_2$ can be removed completely. Since the equilibrium of both of the reactions is shifted towards the right at low temperature, CO and $CO_2$ can, however, be removed to a very high degree when the reaction temperature is maintained low (e.g. about 200°C) and there is employed a catalyst which is particularly active at this low temperature. The residual concentrations of CO and $CO_2$ will, moreover, be the lower the concentrations thereof in the gas mixture subjected to the purification.

Catalysts having activity at low temperature for reactions 4) and 5) and at the same time for the shift reaction 3) mentioned below are well-known. They are mainly copper-containing catalysts. Thus, oxides of copper, chromium and zinc or oxides of copper, zinc and aluminium catalyse these two reactions as well as the shift reaction, even at low temperatures.

Catalysts particularly suitable for the carbon oxides conversion in accordance with the present invention and also the shift reaction are described in Danish patent specification No. 123,810 corresponding to British patent specification No. 1,245,036; and in Danish patent specification No. 115,318.

The catalyst according to the said British specification contains substantially only a mixture of compounds of copper, zinc, chromium and oxygen at least partly in the form of copper chromate $CuCrO_4$ and zinc chromate $ZnCrO_4$ being present in so intimate mixture that they form a common crystal lattice; the catalyst is prepared by dissolving a water-insoluble copper compound in an excess of chromic acid and reacting the resulting solution with a suspension of zinc oxide in an aqueous medium to form a precipitate which is isolated, dried and roasted in the presence of ammonia and then worked up to form catalyst particles.

The catalyst according to Danish patent specification No. 115,318 likewise contains Cu, Zn and Cr and consists of a granular support of zinc chromate where the ratio of ZnO to $CrO_3$ is between 1:1 and 1:3, the support having been impregnated with a copper compound that has been converted into CuO or Cu finely distributed on the granules of the support.

Simultaneously with reactions 4) and 5), the equilibrium between CO and $CO_2$, i.e. of the so called shift reaction adjusts itself at the passage of the gas over the same catalyst bed:

3) $CO + H_2O \quad CO_2 + H_2$

This means that the gas after having passed the catalyst will contain both CO and $CO_2$, but as mentioned, in small concentrations. If the gas mixture prior to purification contains particularly little or possibly nothing of one of these two impurities, more

of this impurity may be present after the passage; but the total content of CO and $CO_2$ will in any case be decreased during the passage of the catalyst.

If the gas mixture before passing the catalyst especially contains very little or no $CO_2$, $CO_2$ will thus be formed and at the same time a corresponding amount of hydrogen will be formed as shown by the reaction scheme. However, the result of the decrease of the total content of CO and $CO_2$ obtained is that hydrogen is removed from the gas mixture according to reaction schemes 4) and 5), a corresponding amount of methanol being formed as shown by the reaction schemes.

Subsequently methanol and water are removed from the gas stream, e.g. by being washed out, and are recycled to be combined with the feed stock of methanol and steam. The formation of hydrogen (with CO and $CO_2$ as by-products) is carried out under such conditions that reactions 4) and 5) proceed in the opposite direction so that CO and $CO_2$ are regenerated and the hydrogen present in combined form in methanol and water is liberated. The major part of the regenerated CO and $CO_2$ is removed in the preliminary gas mixture purification steps, which means that the purification process essentially proceeds without loss of hydrogen, i.e. with a hydrogen output approaching 100%, as will be seen from the Example hereinbelow.

It is necessary that the crude hydrogen-containing gas mixture is first subjected to a preliminary purification to remove a substantial part of CO and/or $CO_2$ before it is subjected to a purification in accordance with the process of the present invention since otherwise CO and/or $CO_2$ will be accumulated in the apparatus in which the process is carried out. However, it is not decisive for the use of the process according to the invention how the preliminary purification is carried out, and neither is it decisive exactly how

much of the content of CO and/or $CO_2$ in the crude gas mixture there is removed before the process according to the invention is put into use, or to which residual concentrations it is utilized. However, the economy of the overall production of pure or almost pure hydrogen will depend upon this because the optimum degree of preliminary purification and the most suitable method of preliminary purification in each individual case depends upon the size of the plant, the composition of the gas and the pressure, and of the prizes of heat and energy per unit thereof. However, according to the invention the content of carbon oxides in the hydrogen containing gas should preferably not exceed 8 mol% at the step of the overall process where they are converted into methanol.

The process according to the invention may be repeated one or more times, i.e. carried out in two or more cycles, it being understood that one cycle comprises catalytic conversion into methanol followed by the removal and recycling of methanol and water to the feedstock. By using a sufficient number of cycles the residual amounts may be brought to a very low level, for which reason economic considerations must be balanced against the desire for degree of purity determined by the ultimate use of the hydrogen.

If the process according to the invention is effected in several cycles one may, if desired, operate the various cycles at different pressures. However, the degree of conversion obtainable increases with increasing pressure, i.e. the residual concentrations decrease, and normally it is therefore advantageous to utilize the process at the highest possible pressure. This particularly is of interest in cases where the use of the hydrogen demands a higher pressure than that at which the preceding process steps are operated - e.g.

the decomposition of methanol and subsequent preliminary purification. In such a case it will normally be advantageous to operate the process according to the invention at or near the pressure at which the hydrogen is to be employed.

The catalytic conversion of CO and $CO_2$ is carried out at temperatures between 100 and 300°C, preferably 150-250°C, which is the temperature range within which catalysts for the desired reactions operate most efficiently. It is an advantage that in the process the hydrogen-containing gas is not admixed with an impurity component which at a temperature of 0-50°C and a pressure of 5-20 $kg/cm^2g$ is single-phase gaseous.

The process according to the invention in the following will be illustrated by the following Example.

Example

The preparation and purification of 50 $Nm^3/h$ $H_2$ is carried out in a plant the fundamental construction of which is shown in the accompanying drawing and described hereinbefore.

The catalyst present in reactor 2 and catalysing reactions 1), 2) and 3) may be of the same type as that catalysing reactions 4) and 5) and described hereinbefore. This, of course, is in accordance with the general principle that a catalyst for any equilibrium reaction catalyzes it in both directions. The catalyst present in the adiabatic reactor 12 to ensure the conversion of CO and $CO_2$ into methanol according to equations 4) and 5) obviously may be of the same kind and so may the catalyst in the adiabatic reactor 4 where the shift reaction 3) is carried out at a lower pressure than in reactor 2.

The catalyst in reactor 17 for catalyzing reactions 6) and 7), which is the reverse reaction of the well-known steam reforming, is preferably a nickel-containing catalyst.

Table 1 below shows data for the various streams.

TABLE

| Stream No. | 1 | 3 | 5 | 8 | 11 | 13 | 16 | 18 | 20 | 22 | 7 | 23 | 15 | 10 | 21 |
|---|---|---|---|---|---|---|---|---|---|---|---|---|---|---|---|
| Pressure, $kg/cm^2g$ | 21 | 20.5 | 20.3 | 20.1 | 19.9 | 19.8 | 19.7 | 19.5 | 19 | 20.3 | - | 19.8 | - | 0 | - |
| Temperature, $^{o}C$ | 275 | 275 | 200 | 30 | 8 | 190 | 12 | 12 | 12 | 8 | - | 8 | - | 8 | - |
| Amount, kmol/h | 2.71 | 4.23 | 4.23 | 3.07 | 2.26 | 2.24 | 2.23 | 2.23 | 2.23 | 0.20 | 1.38 | 0.20 | 0.22 | 0.76 | 0.002 |
| Composition, mol% | | | | | | | | | | | | | | | |
| $H_2$ | - | 53.5 | 54.0 | 74.8 | 99.4 | 99.37 | 99.89 | 99.88 | 99.95 | - | - | - | - | 5.6 | - |
| $H_2O$ | 66.7 | 25.2 | 24.7 | 0.2 | 0.1 | 0.15 | 0.07 | 0.07 | - | 100 | 90.3 | 100 | 95.6 | - | 100 |
| CO | - | 0.8 | 0.2 | 0.3 | 0.4 | 0.03 | 0.03 | - | - | - | - | - | - | - | - |
| $CO_2$ | - | 17.3 | 17.9 | 24.7 | 0.1 | 0.01 | 0.01 | - | - | - | - | - | - | 94.4 | - |
| $CH_4$ | - | - | - | - | - | - | - | 0.05 | 0.05 | - | - | - | - | - | - |
| $CH_3OH$ | 33.3 | 3.2 | 3.2 | - | - | 0.44 | - | - | - | - | 9.7 | - | 4.4 | - | - |

CLAIMS

1. A process for the removal of carbon oxides from a hydrogen-containing gas prepared by the catalytic decomposition of methanol, wherein a preliminary purification of the hydrogen-containing gas thereby produced is carried out partially to remove carbon oxides therefrom, after which the hydrogen-containing gas with reduced carbon oxides content thus obtained is passed at a temperature of 100-300°C and a pressure of 5-300 $kg/cm^2$ gauge over a catalyst for converting carbon oxides into methanol and the methanol formed is removed from the gas, characterised in that the methonol formed is recycled and combined with the methanol used as raw material for preparing the hydrogen-containing gas.

2. A process as claimed in claim 1, characterised in that the catalytic conversion of the carbon oxides present in the hydrogen-containing gas is carried out at a temperature of 150-250°C and at a pressure of 15-150 $kg/cm^2$ gauge.

3. A process as claimed in claims 1 or 2, characterised in that the content of carbon oxides in the hydrogen-containing gas when converting them into methanol does not exceed 8 mol%.

4. A process as claimed in any of claims 1 to 3, characterised in purifying further the hydrogen-containing gas for carbon oxides by repeating one or more times the catalytic conversions of carbon oxides into methanol and recycling the latter to the raw material for preparing the hydrogen.

5. A process as claimed in any of claims 1 to 4, characterised in that the catalyst used for converting the carbon oxides into methanol is a known catalyst consisting substantially only of compounds of copper, zinc, chromium and oxygen and prepared by dissolving a water-insoluble copper compound in an excess of chromic acid, reacting the resulting solution with a

suspension of zinc oxide in an aqueous medium to form a precipitate which is isolated, dried, roasted in the presence of ammonia and worked up to form catalyst particles.

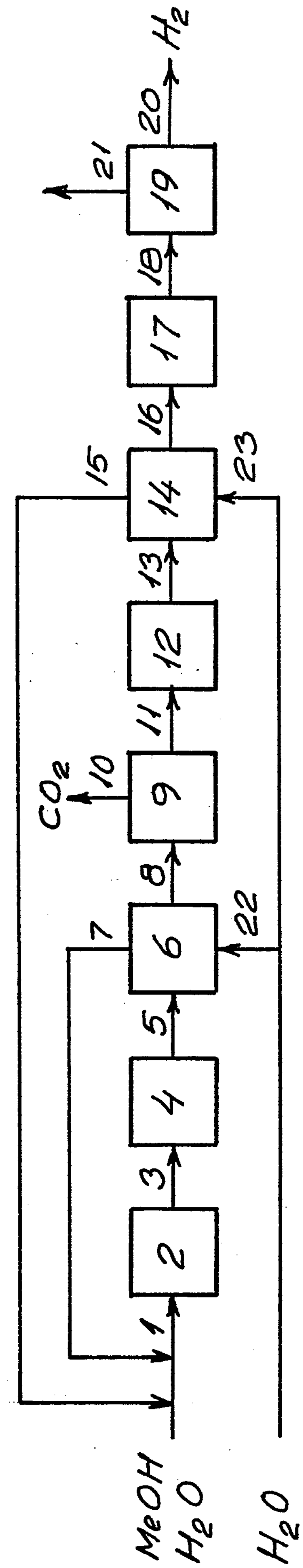

MeOH
$H_2O$
$H_2O$
1
2
3
4
5
6
7
22
8
9
10
$CO_2$
11
12
13
14
15
23
16
17
18
19
21
20
$H_2$